# EUROPEAN PATENT APPLICATION

(11) **EP 4 596 031 A1**
(43) Date of publication of application: **06.08.2025**
(21) Application number: 23922313.4
(22) Date of filing: 28.09.2023
(51) Int. Cl.: A61N 1/39

(54) **DEFIBRILLATION DEVICES**

(30) Priority: 17.02.2023 WO PCT/CN2023/076919
(71) Applicant: Shenzhen Mindray Bio-Medical Electronics Co., Ltd., Shenzhen, Guangdong 518057 (CN)
(72) Inventor: CHEN, Da Bing, Shenzhen, Guangdong 518057 (CN); HE, Zi Rui, Shenzhen, Guangdong 518057 (CN); XU, Li, Shenzhen, Guangdong 518057 (CN)
(74) Representative: KIPA AB
(86) International application number: PCT/CN2023/122825
(87) International publication number: WO 2024/169195

(57) **Abstract**

Defibrillation devices (100). A defibrillation device comprises a defibrillation assembly (110), a first processor (120) and a second processor (130). The defibrillation assembly (110) is used for executing defibrillation tasks; the first processor (120) acquires data from the defibrillation assembly (110) and processes same to obtain defibrillation data; the second processor (130) acquires expansion device data from an expansion device (10); the second processor (130) is connected to the first processor (120); and the second processor (130) and the first processor (120) can transmit preset information. After the defibrillation device (100) is started, the first processor (120) is in a working state by default, and the second processor (130) is in an off state or a low-power-consumption state by default; and the second processor (130) can be switched to a working state, so that the defibrillation devices (100) can quickly complete startup to execute basic defibrillation tasks, thereby satisfying the first-aid requirements of some scenarios. In addition, the interference of the second processor (130) and the expansion device (10) on the first processor (120) and the defibrillation assembly (110) can also be prevented, thereby ensuring the reliability of the defibrillation devices (100) executing defibrillation tasks.

## Description

### TECHNICAL FIELD

The disclosure relates to a technical field of medical device, and more particularly to a defibrillation device.

### BACKGROUND

Electric shock defibrillation is the only effective treatment for sudden cardiac arrest (SCA), and a defibrillation monitor is a necessary device for electric shock defibrillation. Currently, the defibrillation monitor is hoped to integrate more functions, so as to enable a pre-hospital emergency care to cope with a situation, such as an internal bleeding situation, and a large-scale disaster scene. However, integrating more functions will prolong a starting time of the defibrillation monitor, or may also affect stability and rescue.

### SUMMARY

This disclosure provides a defibrillation device, aiming to solve a technical problem, such as a prolonged starting time or an affected stability resulted by integrating more functions in a defibrillation device.

In a first aspect, an embodiment of this disclosure provides a defibrillation device, including:
a defibrillation assembly, which is configured to perform a defibrillation task;
a first processor, which is configured to acquire data from the defibrillation assembly and process the data to obtain defibrillation data; and
a second processor, which is configured to acquire extension device data from an extension device;
wherein the second processor is connected with the first processor, the second processor and the first processor are capable of transmitting preset information;
after the defibrillation device is started, the first processor is in a working state by default, the second processor is in a turn-off state or a low power consumption state by default, wherein the second processor is capable of being switched to a working state.

In a second aspect, an embodiment of this disclosure provides a defibrillation device, including:
a defibrillation assembly, which is configured to perform a defibrillation task;
a first processor, which is configured to acquire data from the defibrillation assembly and process the data to obtain defibrillation data; and
a second processor, which is configured to acquire extension device data from an extension device and/or extension monitoring data from an extension monitoring assembly;
wherein the second processor is connected with the first processor, the second processor and the first processor are capable of transmitting preset information;
after the defibrillation device is started, the first processor is in a working state by default, the second processor is capable of being in a turn-off state, a low power consumption state or a working state; wherein, when the second processor does not acquire the extension device data or the extension monitoring data, a duration when the second processor is in the working state is less than a duration when the second processor is in the turn-off state and/or the low power consumption state.

In a third aspect, an embodiment of this disclosure provides a defibrillation device, including:
a defibrillation assembly, which is configured to perform a defibrillation task;
a first processor, which is configured to acquire data from the defibrillation assembly and process the data to obtain defibrillation data; and
a second processor, which is configured to acquire extension device data from an extension device;
wherein the second processor is connected with the first processor, the second processor and the first processor are capable of transmitting preset information;
after the defibrillation device is started, the first processor is in a working state by default, the second processor is capable of being in a turn-off state, a low power consumption state or a working state; wherein a duration when the second processor is in the working state is less than a duration when the first processor is in the working state.

The defibrillation device provided in an embodiment of this disclosure includes a defibrillation assembly, a first processor and a second processor; wherein the defibrillation assembly is configured to perform a defibrillation task; the first processor is configured to acquire data from the defibrillation assembly and processes said data to obtain defibrillation data; the second processor is configured to acquire extension device data from an extension device; the second processor is connected with the first processor, the second processor and the first processor are capable of transmitting preset information; after the defibrillation device is started, the first processor is in a working state by default, the second processor is in a turn-off state or a low power consumption state by default, wherein the second processor is capable of being switched to a working state. In this way, the defibrillation device is capable of quickly and completely starting to perform a basic defibrillation task and satisfying an emergency requirement of some scenarios. Furthermore, interference on the first processor and the defibrillation assembly from the second processor and the extension device can be prevented, which ensures a reliability of the defibrillation device in performing a defibrillation task.

It should be understood that the above general description and the following detailed description are merely exemplary and explanatory and cannot limit a disclosed content of embodiments of this disclosure.

### BRIEF DESCRIPTION OF THE DRAWINGS

In order to more clearly illustrate technical solutions of embodiments of this disclosure, drawings required for use in the description of the embodiments will be briefly introduced below. Obviously, the drawings described below are some embodiments of this disclosure. For ordinary technicians in this field, other drawings can be obtained based on these drawings without paying any creative work.
FIG. 1 is a structural diagram of a defibrillation device provided in an embodiment of this disclosure.
FIGS. 2~7 are structural diagrams of defibrillation devices in different embodiments.
FIG. 8 is a schematic diagram of a working portion of a defibrillation device when started in one embodiment.
FIGS. 9~10 are schematic structural diagrams of defibrillation devices in other embodiments.

Explanation of reference numbers:
100, defibrillation device; 110, defibrillation assembly; 111, defibrillation function assembly; 112, pacing function assembly; 120, first processor; 130, second processor; 140, monitoring assembly; 141, ECG detection assembly; 142, blood oxygen detection assembly; 143, NIBP detection assembly; 150, communication assembly; 160, human-machine interaction assembly; 10, extension device; 11, ultrasound device; 12, laryngoscope device; 20, extension function assembly; 30, extension monitoring assembly; 170, power supply apparatus; 101, isolation circuit; 102, switching assembly.

### DESCRIPTION OF THE PREFERRED EMBODIMENTS

Technical solutions in embodiments of this disclosure will be clearly and completely described below in conjunction with the drawings in the embodiments of this disclosure. Obviously, the described embodiments are only part of embodiments of this disclosure, rather than all of the embodiments. Based on the embodiments in this disclosure, all other embodiments obtained by ordinary technicians in this field without making any creative work shall fall within the scope of protection of the disclosure.

The flowcharts shown in the drawings are only examples and do not necessarily include all contents and operations/steps, nor do they necessarily have to be executed in the order described. For example, some operations/steps may be decomposed, combined or partially merged, so that an actual execution order may change according to actual circumstances.

Some implementation methods of this disclosure are described in detail below in conjunction with the accompanying drawings. In the absence of conflict, the following embodiments and features of the embodiments may be combined with each other.

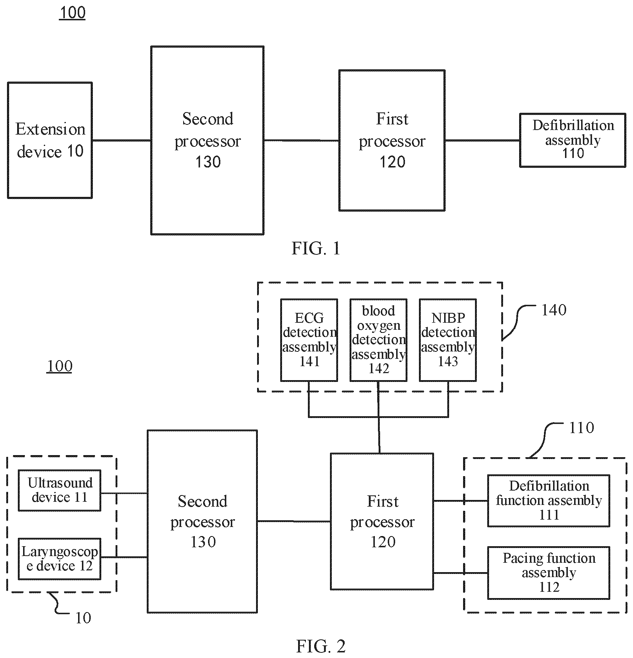

Please refer to FIG. 1, which is a block diagram of a defibrillation device 100 provided in an embodiment of this disclosure.

The defibrillation device 100 is a high-risk first-aid device, which is mainly used for defibrillation treatment of dangerous diseases, such as ventricular fibrillation and atrial fibrillation. Optionally, the defibrillation device 100 includes at least one of: an ambulance defibrillator, an in-hospital emergency defibrillator, an automatic external defibrillator (AED). Of course, the defibrillation device 100 is certainly not limited thereto.

As shown in FIG. 1, the defibrillation device 100 includes a defibrillation assembly 110, a first processor 120, and a second processor 130.

The defibrillation assembly 110 is configured to perform a defibrillation task.

Exemplarily, the defibrillation assembly 110 includes a charge-discharge circuit, which is configured to boost up a low-voltage DC electrical power and store the boosted power in an energy storage capacitor, and to release the power stored in the energy storage capacitor to output a defibrillation waveform.

For example, the defibrillation assembly 110 is connected with or is capable of being connected with a defibrillator electrode, and the charge-discharge circuit performs a discharge treatment to a patient body through the defibrillator electrode according to an instruction of the first processor 120. For example, when the defibrillation assembly 110 performs a defibrillation task, it at least controls the charge-discharge circuit to release the electric energy stored in the energy storage capacitor and outputs a defibrillation waveform.

In some embodiments, referring to FIG. 2, the defibrillation device 100 further includes a monitoring assembly 140, and the first processor 120 is further configured to acquire data from the monitoring assembly 140 and process the data to obtain monitoring data.

Exemplarily, the monitoring assembly 140 includes at least one of: an electrocardiogram detection assembly 141, a blood oxygen detection assembly 142, and an automated non-invasive blood pressure (NIBP) detection assembly 143.

Exemplarily, the monitoring assembly 140 is configured to obtain monitoring data of the patient, such as a physiological data signal, including at least one of: an electrocardiogram (ECG) signal, a blood oxygen saturation signal, a non-invasive blood pressure signal, an arterial blood pressure signal, a body temperature signal, a heart rate signal, a respiratory impedance signal, of course, the physiological data signal is not limited to these.

For example, the monitoring assembly 140 is connected with or is capable of being connected with an electrode sheet, such as a disposable ECG electrode sheet. The ECG detection assembly 141 is configured to acquire an ECG signal through the electrode sheet attached to the patient body, and process the ECG signal to obtain at least one of: a heart rate, a PR interval, a QRS interval, etc. The monitoring assembly 140 is connected with or is capable of being connected with a blood oxygen probe, and is capable of determining blood oxygen saturation of a patient based on a signal outputted by the blood oxygen probe. Of course, it is not limited to this. For example, the monitoring assembly 140 can transmit a digital signal outputted by the blood oxygen probe to the first processor 120, which digital signal indicates the blood oxygen saturation.

In some embodiments, the first processor 120 is configured to obtain monitoring data through the monitoring assembly 140 and to perform a defibrillation task through the defibrillation assembly 110.

Exemplarily, the first processor 120 is configured to control the defibrillation assembly 110 to perform a defibrillation task according to the monitoring data. For example, the defibrillation task includes a biphasic defibrillation, but is certainly not limited thereto. The monitoring assembly 140, such as an ECG electrode sheet, is brought into contact with a suitable part of a patient body (e.g., human body), and the monitoring assembly 140 acquires and analyzes treatment-related information. For example, the monitoring assembly 140 measures impedance information of a contact circuit between the electrode sheet and the human body; determines whether the electrode sheet and the human body are in good contact and whether the patient is moving, by detecting human body impedance; uses such information as a reference for an adjustment parameter of subsequent biphasic wave defibrillation; detects whether the patient has a built-in pacemaker, and when the patient is detected to have a built-in pacemaker, obtains a Pace mark of the pacemaker; acquires ECG (electrocardiogram) waveform data of human body through the ECG electrode sheet. The first processor 120 combines above analysis results of the contact impedance, Pace signal and ECG waveform data to determine whether the patient is ready and has a defibrillation rhythm. If a defibrillation rhythm is detected, the defibrillation assembly 110 is started to treat the patient.

Exemplarily, the defibrillation assembly 110 includes a defibrillation function assembly 111 and/or a pacing function assembly 112. Wherein, the defibrillation function assembly 111 is mainly aimed at a malignant arrhythmia, such as pulseless ventricular tachycardia and ventricular fibrillation. Through defibrillation, the arrhythmia that may be life-threatening can be treated, so as to save a life of patient and effectively prevent sudden death. The pacing function assembly 112 is mainly aimed at a patient with a slower heart rate, such as a patient with a problem in a cardiac conduction system. No matter for a patient with a problem in a sinoatrial node function, in a cardiac pacing, or in an atrioventricular node, the heart can be stimulated to cause an effective cardiac pacing. If the patient has a problem in the atrioventricular node, the pacing function assembly 112 can also replace the atrioventricular node for conduction. It can be understood that the defibrillation function assembly 111 is mainly used for a termination treatment of arrhythmia; the pacing function assembly 112 is mainly used for maintain a heartbeat of a patient with a slower heart rate to be within a normal range.

The first processor 120 is configured to acquire data from the defibrillation assembly 110 and processes the data to obtain defibrillation data. For example, the first processor 120 obtains from the defibrillation assembly 110 at least one of energy for defibrillation and a time of electric shock for defibrillation, and processes the obtained defibrillation data. Of course, it is not limited thereto.

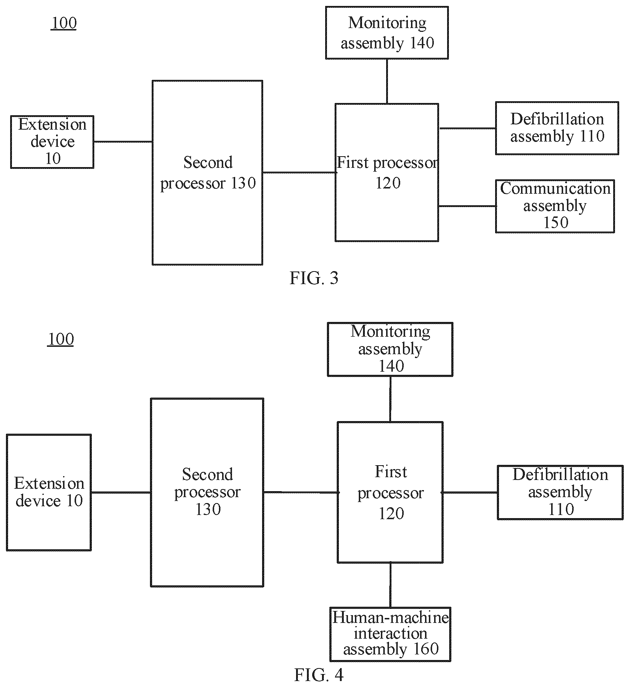

In some embodiments, referring to FIG. 3, the defibrillation device 100 further includes a communication assembly 150. As shown in FIG. 3, the communication assembly 150 is connected with the first processor 120, and can implement a network function when the defibrillation device 100 is started. The communication assembly 150 may be used to implement an external communication function of the defibrillation device 100. For example, the first processor 120 is also configured to establish a communicative connection with a target device through the communication assembly 150.

Exemplarily, the first processor 120 is configured to transmit defibrillation data and/or monitoring data to the target device through the communication assembly 150; the monitoring data is obtained by the first processor 120 through processing the data obtained from the monitoring assembly 140. The target device can store, display, process, make decision(s), etc. on the defibrillation data and/or the monitoring data.

Exemplarily, the first processor 120 is further configured to obtain control data from the target device through the communication assembly 120, and control the defibrillation assembly 110 according to the control data. For example, the target device may determine corresponding control data based on the defibrillation data and/or the monitoring data to provide remote guidance to a user at an emergency scene.

For example, the target device includes at least one of a server and a terminal device, wherein the target device and/or a user of the target device can provide remote guidance based on the monitoring data. Of course, it is not limited to this. For example, the target device has a display with a larger area and/or a higher resolution, which can display the data transmitted by the first processor 120 better, making it easier for user operation.

Exemplarily, the first processor 120 can be connected with the network server in real time through the communication assembly 150, when the defibrillation device 100 is started, so that after the defibrillation device 100 is started, the defibrillation device 100 can work according to a requirement of the network server.

Exemplarily, the communication assembly 150 may include at least one of: a video transmission interface, a network cable interface, a USB interface, and a wireless communication assembly; wherein the video transmission interface includes, for example, a high-definition multimedia interface (HDMI), the wireless communication assembly includes, for example, a 5G (5th Generation Mobile Communication Technology) assembly, but is certainly not limited thereto, and may include, for example, a 4G (4th Generation Mobile Communication Technology) assembly.

Specifically, referring to FIG. 1 and FIG. 2, the second processor 130 is connected with the first processor 120, and the second processor 130 and the first processor 120 are capable of transmitting preset information. In some implementations, the second processor 130 transmits the preset information to the first processor 120 unidirectionally. In other implementations, the preset information can be transmitted bidirectionally between the first processor 120 and the second processor 130. It can be understood that data and/or instructions are transmitted between the first processor 120 and the second processor 130 of the defibrillation device 100, so as to achieve a coordination of the defibrillation function, the monitoring function and the extension function, that is, the defibrillation device 100 is multifunctional integrated.

In some implementations, the second processor 130 may perform data transmission with the target device through the first processor 120 and the communication assembly 150. In other embodiments, the defibrillation device 100 further includes an extension communication assembly which is connected with the second processor 130, and data is capable of being transmitted between the second processor 130 and the target device through the extension communication assembly. A target device, which is in communication with the defibrillation device 100 through the communication assembly 120, and a target device, which is in communication with the defibrillation device 100 through the extension communication assembly, may be a same target device or different target devices.

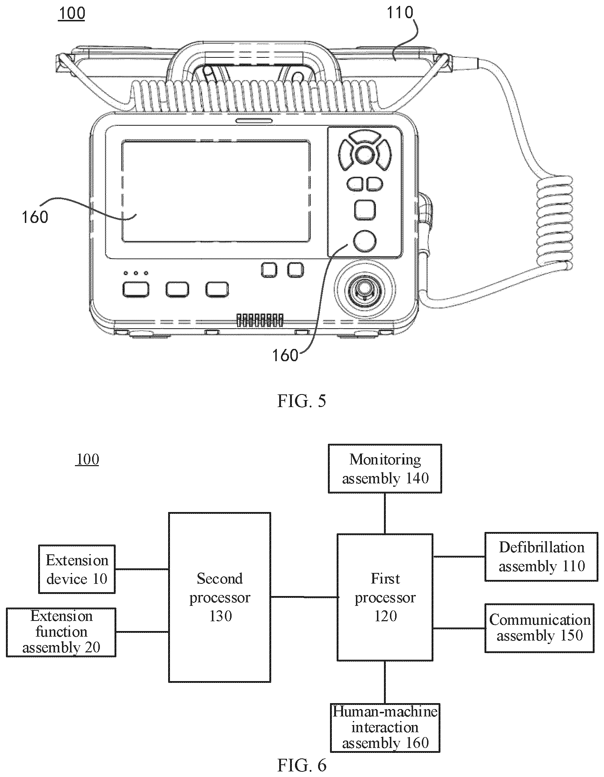

In some embodiments, as shown in FIGS. 4 and 5, the defibrillation device 100 further includes a human-machine interaction assembly 160. Optionally, the human-machine interaction assembly 160 is connected with the first processor 120. Illustratively, the first processor 120 outputs defibrillation data and/or monitoring data through the human-machine interaction assembly 160. When the human-machine interaction assembly 160 is connected with the first processor 120, the human-machine interaction assembly 160 can realize at least part of a human-machine interaction function corresponding to the defibrillation function and the monitoring function. Of course, it is not limited to this. For example, the human-machine interaction assembly 160 can also be connected with the second processor 130. When the second processor 130 is in a low power consumption state or a working state, it can output data or obtain an instruction through the human-machine interaction assembly 160; or when the second processor 130 is in a working state, the first processor 120 is also configured to output data transmitted by the second processor 130 through the human-machine interaction assembly 160.

Exemplarily, the human-machine interaction assembly 160 includes at least one of: a display assembly, a mouse, a keyboard, a touch pad, a touch display assembly, a button, a knob, a microphone, a speaker, and a prompt light. For example, the first processor 120 displays the defibrillation data and/or the monitoring data through a display interface of the human-machine interaction assembly 160. It should be noted that some human-machine interaction assemblies 160, such as a mouse, can be detachably connected with a main body of the defibrillation device 100.

The second processor 130 is configured to obtain extension device data from an extension device 10. Specifically, when the second processor 130 is in a working state, the second processor 130 at least obtains the extension device data from the extension device 10. In some embodiments, referring to FIG. 2, the extension device 10 includes an ultrasound device 11 and/or a laryngoscope device 12.

Wherein, the ultrasound device 11 is capable of emitting ultrasound to the target object, receiving an ultrasound echo of the ultrasound returned from the target object to acquire an ultrasound echo signal, and generating an ultrasound image based on the ultrasound echo signal. Optionally, the ultrasound device 11 is capable of emitting ultrasound to the target object, receiving an ultrasound echo of the ultrasound returned from the target object to acquire an ultrasound echo signal. It can be understood that the ultrasound device 11 may include a complete ultrasound imaging device, or may only include an ultrasound echo signal acquisition device, such as an ultrasound probe.

Wherein, the laryngoscope device 12 is, for example, an electronic laryngoscope, which includes, for example, an imaging head, and may also include a light source. An examination scope of the electronic laryngoscope includes a nasal cavity, a nasopharynx, an oropharynx, a hypopharynx, and a larynx, and can even penetrate into a trachea to understand a condition of the trachea, providing a clearer basis for inner and lower boundaries of laryngeal tumor lesions.

Exemplarily, acquiring, by the second processor 130, the extension device data from the extension device 10, including: acquiring, by the second processor 130, ultrasound data and/or laryngoscope data from the ultrasound device 11 and/or laryngoscope device 12. For example, the ultrasound data is ultrasound image data, such as an ultrasound image generated by the ultrasound device 11, and/or the laryngoscope data is laryngoscope image data. Of course, it is not limited thereto. For example, the ultrasound data is an ultrasound echo signal acquired by the ultrasound device 11.

Exemplarily, the second processor 130 may process the acquired extension device data. For example, the second processor 130 acquires the ultrasonic echo signal from the ultrasonic device 11, and generates an ultrasonic image based on the ultrasonic echo signal. The second processor 130 can process the ultrasonic echo signal to obtain a corresponding ultrasonic image. For example, the second processor 130 has a stronger performance, for example, at least stronger than the first processor 120, and can execute more complex algorithms, such as image processing.

In some embodiments, referring to FIG. 6, the defibrillation device 100 further includes an extension functional assembly 20 which is connected with the second processor 130.

Exemplarily, the extension function assembly 20 includes at least one of: a data reading assembly, an extension human-machine interaction assembly, and a photographing assembly.

The data reading assembly includes at least one of: a magnetic stripe card reading assembly, a chip card reading assembly, and a near field communication (NFC) assembly, but is certainly not limited thereto.

Exemplarily, the second processor 130 acquires the extension device data through at least one of: a data reading assembly, an extension human-machine interaction assembly, and a photographing assembly, and transmits the extension device data to the target device and/or the first processor 120.

Exemplarily, the user may input an operation instruction for acquiring and transmitting the extension device data through the extension human-machine interaction assembly, and the second processor 130 may transmit the extension device data to the target device and/or the first processor 120 according to the operation instruction.

Exemplarily, the second processor 130 may read data stored in a preset device through a data reading assembly, and/or may photograph an image by a photographing assembly. For example, the second processor 130 reads, through the data reading assembly, data of an identity card, a social security card, etc.; or photographs, through the photographing assembly, an image of a patient or a part of patient body, or a scene image of an emergency scene. Optionally, the second processor 130 reads the data stored in the preset device, such as a medical history, medication state, etc., of the patient, and said data can be used as a reference for the first processor 120 when performing a defibrillation task.

Exemplarily, the second processor 130 may also transmit the data read by the data reading assembly and/or the image photographed by the photographing assembly to the target device, and obtain feedback information transmitted by the target device based on the data and/or image. For example, the second processor 130 transmits the data read from the social security card to the server, and obtains feedback information transmitted by the server, wherein the feedback information includes, for example, at least one of: a medical history and a medication state of the patient. For example, the second processor 130 transmits an image of a patient or a part of a patient or a scene image to a target device, so that the target device and/or a user of the target device can provide remote guidance or arrange first-aid treatment.

It can be understood that the second processor 130 is further configured to obtain feedback information transmitted by the target device in response to the extension device data. Exemplarily, the target device may automatically determine feedback information according to the extension device data and transmit the feedback information, or may output the extension device data to a user, determine feedback information according to a user operation, and transmit the feedback information.

For example, the second processor 130 is further configured to transmit the feedback information to the first processor 120, and the first processor 120 outputs the feedback information through the human-machine interaction assembly 160, so as to guide a user of the defibrillation device 100, or output information required by a user.

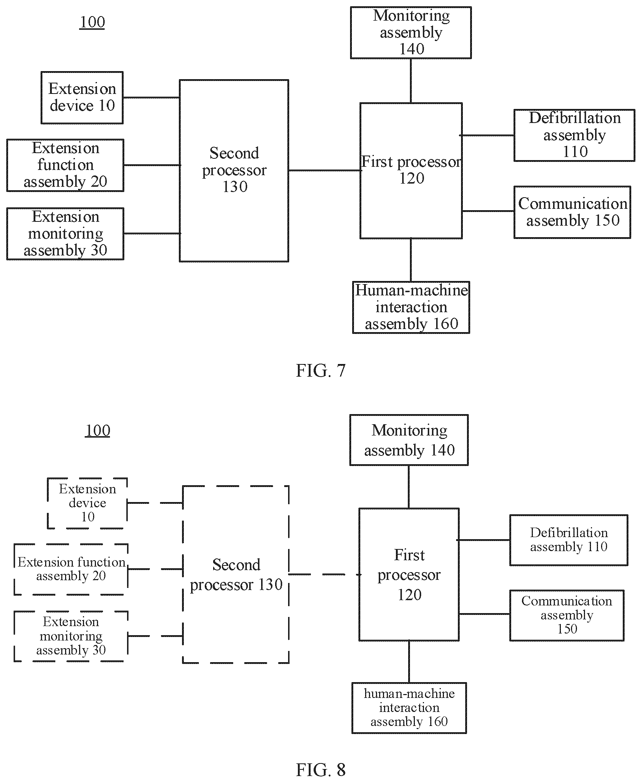

In some embodiments, referring to FIG. 7, the defibrillation device 100 further includes an extension monitoring assembly 30, and the second processor 130 is further configured to obtain extension monitoring data from the extension monitoring assembly 30 when in a working state.

Exemplarily, the extension monitoring assembly 30 can be configured to obtain monitoring data different from the aforementioned monitoring assembly 140, such as at least one of: carbon dioxide (CO2), body temperature, respiratory impedance, etc.. Optionally, the extension monitoring assembly 30 includes a body temperature sensor, etc. Of course, this disclosure is not limited thereto. For example, the extension monitoring assembly 30 can obtain the same monitoring data as the aforementioned monitoring assembly 140.

Exemplarily, the second processor 130 is configured to acquire data from the extension monitoring assembly 30, or transmit the processed data to the first processor 120, and the first processor 120 is configured to output the processed data through the human-machine interaction assembly 160, or the first processor 120 is configured to perform a defibrillation task through the defibrillation assembly 110 according to said data. The types of monitoring data can be flexibly extended by extending the monitoring assembly 30 according to the usage scenario, thereby improving the accuracy of the first processor 120 when performing defibrillation through the defibrillation assembly 110.

In some embodiments of this disclosure, after the defibrillation device 100 is started, the first processor 120 is in a working state by default, the second processor 130 is in a turn-off state or a low power consumption state by default, and the second processor 130 is capable of being switched to a working state. A state by default may be a state preset when the defibrillation device 100 leaves the factory, or may be selected and set by the user from a variety of optional configurations. In some embodiments, the state by default may include a situation in which a user has not performed any operation other than starting the device, and the defibrillation device 100 is in a normal operating state. In other embodiments, the state by default also include a situation in which only a non-first-aid operation is performed on the defibrillation device 100. The first aid operation, for example, includes: using the defibrillation device 100 to implement a defibrillation function, a monitoring function, etc., and its direct purpose is to provide first aid to the patient. Non-first-aid operation, for example, includes: adjusting a screen brightness, adjusting a volume, switching a language, etc., whose direct purpose is to adjust a configuration of the defibrillation device 100, etc., and only indirectly affect the first-aid treatment of the patient, or even have no effect on the first-aid treatment of the patient. In other embodiments, the state by default may include a situation in which a following extension start condition is not satisfied or a following extension start instruction is not received.

In some implementations, when the defibrillation device 100 has been completely started, the first processor 120 is switched from the turn-off state to the working state, and the second processor 130 remains in the turn-off state or is switched to the low power consumption state. The defibrillation device 100 having been completely started means that the user can operate the defibrillation device 100 through the human-machine interaction assembly 160, such as buttons or a touch screen. It can be understood that in other embodiments of this disclosure, when the defibrillation device 100 has been completely started, the first processor 120 is switched from the turn-off state to the working state, and the second processor 130 is in the turn-off state or the low power consumption state.

For example, referring to FIG. 8, portions of the defibrillation device 100 that work, when the defibrillation device 100 is started, include the defibrillation assembly 110, the first processor 120, the monitoring assembly 140, the human-machine interaction assembly 160 and the communication assembly 150. When the defibrillation device 100 is started, the first processor 120 is switched to a working state. In the working state, the first processor 120 acquires data from the defibrillation assembly 110 and processes the data to obtain defibrillation data, so that the defibrillation device 100 can at least realize the defibrillation function through the first processor 120, and can also realize a monitoring function and a network function to satisfied a basic requirement of the defibrillation device 100 to perform a defibrillation task. Even if the second processor remains in the turn-off state, the defibrillation device 100 can also perform a basic defibrillation task. When the defibrillation device 100 is started, the second processor 130 remains in the turn-off state, so that the defibrillation device 100 can quickly complete starting, can perform a basic defibrillation task, and satisfy a first-aid requirement of some scenarios.

The defibrillation device 100 of the embodiment of this disclosure adopts a multi-processor architecture. The first processor 120 may serve as a main processor and is mainly responsible for conventional defibrillation monitoring functions and tasks, including, for example, a defibrillation function, a monitoring function (such as ECG acquisition and algorithm analysis), a network function, and the likes. The functions of the first processor 120 are core systems of the defibrillation device 100, which can basically satisfy the conventional in-hospital and ambulance usage scenarios.

The extension functions of the extension device 10 are implemented through the second processor 130. The second processor 130 can be used as an extension processor to be responsible for some external extension functions, such as ultrasound, imaging head and other extension functions, so as to achieve functional isolation between the defibrillation function, the monitoring function and the extension function; wherein the extension functions of the extension device 10 can be continuously extended according to clinical needs. It can be determined that the defibrillation device 100 has strong scalability.

The malfunction of the second processor 130 or the malfunction of the extension device 10 never affects abilities of the first processor 120 to acquire data from the defibrillation assembly 110, process the data for obtaining defibrillation data, and never affects an ability of the first processor 120 to implement a core defibrillation rescue function, which has a high security. Even if the second processor 130 or the extension device 10 malfunctions while the defibrillation device 100 is started, the defibrillation device 100 can quickly complete starting and perform a basic defibrillation task to satisfy the first-aid requirements of some scenarios.

Respective system of the second processor 130, the extension device 10, the extension function assembly 20, and the extension monitoring assembly 30 is sometimes unstable or has low reliability. At the same time, due to the powerful performance and high power consumption, their system interference and other problems are relatively large. The second processor 130 is maintained in the turn-off state while the defibrillation device 100 is started, and the extension device 10 may not work, so as to prevent the extension device 10 and the second processor 130 from interfering with the first processor 120 and the defibrillation assembly 110, thereby ensuring the reliability of the defibrillation device 100 in performing a defibrillation task and a monitoring task. When the extension device 10 is not working normally, it can be turned off or put into a standby mode, and when needed, the second processor 130 can be controlled to be switched to a working state, which can greatly reduce system power consumption and improve system reliability. Especially in the pre-hospital scenario, the defibrillation device 100 of the embodiment of this disclosure can satisfy requirements of reliability and standby time.

In comparison, a processor of a traditional defibrillation device is difficult to update quickly due to safety and stability requirements, so its performance is limited. Adding new applications or functions on the basis of monitoring and defibrillation requires a lot of evaluation, and the extension functions cannot be too complex. When the defibrillation device 100 of the embodiment of this disclosure is started, the second processor 130 remains in a turn-off state, that is, the externally extended processing system does not need to perform the defibrillation function and is basically not limited by a stability requirement of the defibrillation device. A new processor with a higher performance can be used, and can be updated and extended at any time, and performance improvement is also easier.

It should be noted that the defibrillation device is a first-aid device and has an extremely high device safety requirement. Traditional defibrillator processor can satisfy the requirement when handling a defibrillation monitoring task, but if complex functions are continuously extended and enriched, a single processor will have an extremely high risk. Its performance may not be able to satisfy requirements of complex task systems and complex software management, and a product risk will be greatly increased. The defibrillation device 100 of the embodiment of this disclosure uses a multi-processor architecture, including a first processor 120 specifically responsible for a defibrillation function, a monitoring function, and a network function, and a second processor 130 responsible for extending complex functions that require large amounts of data processing. Such a multi-processor architecture can effectively solve such problems. Moreover, the first processor 120 alone can satisfy a basic requirement of the defibrillation device 100 to perform a defibrillation task. The second processor 130 is maintained in a turn-off state when the defibrillation device 100 is started. Under normal circumstances, the second processor 130 does not need to work, so that the defibrillation device 100 has lower power consumption and can support the execution of defibrillation task and monitoring task for a longer time, thus achieving a balance between performance and power consumption.

In some embodiments, power consumption of the first processor 120 when in a working state is less than power consumption of the second processor 130 when in a working state; and/or a performance parameter of the first processor 120 is less than a performance parameter of the second processor 130; wherein the performance parameter includes at least one of: a number of core, a main frequency, a cache. The first processor 120 uses a mature, reliable, low-power consumption processor, which can reduce the probability of malfunction and ensure rapid starting of the defibrillation device 100, so as to start quickly during rescue for satisfying the demand of every second. The second processor 130 is responsible for extending complex functions. The clinical risks of these functions are relatively low, but they require the processing of a large amount of data, such as video processing, which involves complex algorithms and requires a strong computing and storage performance. The second processor 130 can use a processor with a stronger performance parameter and/or power consumption.

Exemplarily, the second processor 130 can be selectively disassembled from the defibrillation device 100. Since the first processor 120 alone can satisfy a basic requirement of the defibrillation device 100 to perform a defibrillation task, after the second processor 130 is disassembled, the defibrillation assembly 110 is still capable of performing a defibrillation task. For example, the second processor 130 and the first processor 120 are respectively assembled on different circuit boards. For example, the first processor 120 is assembled on a first circuit board, and the second processor 130 is assembled on a second circuit board; wherein the second circuit board can be selectively disassembled from the defibrillation device 100, and after the second circuit board is disassembled, the defibrillation assembly 110 is still capable of performing the defibrillation task. For example, the second processor 130 and at least some components of the extension device 10 are assembled on the second circuit board; the first processor 120 and at least some components of the defibrillation assembly are assembled on the first circuit board. The second processor 130 is optional. For example, the defibrillation device 100 equipped with the second processor 130 and a corresponding extension device 10 may be configured in a highly professional medical institution, while some models of defibrillation device 100 may not be equipped with the second processor 130, thereby reducing system costs. Furthermore, it is convenient to continuously upgrade the software and hardware of the second processor 130. For example, the second processor 130 may adopt a processor of the latest technology.

In some implementations, when the first processor 120 is in a working state, the second processor 130 is in any one of following processor states: a turn-off state, a low power consumption state, and a working state. When the first processor 120 controls the defibrillation assembly 110 to perform a defibrillation task, the second processor 130 will not affect the execution of the defibrillation task.

In some other embodiments of this disclosure, after the defibrillation device 100 is started, the first processor 120 is in the working state by default, the second processor 130 may be in the turn-off state, the low power consumption state or the working state, and a duration when the second processor 130 is in the working state is less than a duration when the first processor 120 is in the working state. Specifically, after the defibrillation device 100 is started, the second processor 130 may be in the turn-off state or the low power consumption state, so that the duration when the second processor 130 is in the working state is less than the duration when the first processor 120 is in the working state; or after the defibrillation device 100 is started, the second processor 130 is in the working state for a time period and then switched to the turn-off state or the low power consumption state, so that the duration when the second processor 130 is in the working state is less than the duration when the first processor 120 is in the working state. That is, when the defibrillation device 100 is operating normally, since the first processor 120 is in the working state by default, by controlling the second processor 130 to be in the turn-off state or the low power consumption state instead of the working state for at least at some time, the duration when the second processor 130 is in the working state can be less than the duration when the first processor 120 is in the working state, which can prevent the second processor 130 and the corresponding extension device 10 from interfering with the first processor 120, thereby ensuring the reliability of the defibrillation device when performing the defibrillation task, and also reducing the power consumption of the whole device. For example, no matter what operation a user performs on the extension device 10, the extension function assembly 20, and the extension monitoring assembly 30, the execution of the defibrillation task will not be affected.

In some other embodiments of this disclosure, after the defibrillation device 100 is started, the first processor 120 is in the working state by default, and the second processor 130 may be in the turn-off state, the low power consumption state or the working state, and when the second processor 130 does not acquire extension device data or extension monitoring data, the duration when the second processor 130 is in the working state is less than the duration when the second processor 130 is in the turn-off state and/or the low power consumption state. Similarly, by reducing the duration when the second processor 130 is not actually working but is still in the working state, the second processor 130 and the corresponding extension device 10 can be prevented from interfering with the first processor 120, thereby ensuring the reliability of the defibrillation device when performing the defibrillation task, and also reducing the power consumption of the entire device.

In some embodiments, when the second processor 130 is in the working state, the second processor 130 is further configured to acquire at least extension device data from the extension device 10, and may be further configured to acquire data from the extension function assembly 20 and/or the extension monitoring assembly 30, and may further be configured to implement data transmission with the first processor 120, for example, transmitting the acquired extension device data to the first processor 120 or transmitting the acquired extension device data to the first processor 120 after processing. The second processor 130 stops acquiring the extension device data from the extension device 10, when the second processor 130 is in the turn-off state or the low power consumption state.

Exemplarily, when the second processor 130 is in the working state, the second processor 130 is further configured to transmit the acquired or processed extension device data to the target device through the communication assembly 150 or the extension communication assembly. For example, when the second processor 130 is in the working state, the second processor 130 is further configured to generate target visual information according to the defibrillation data and/or the monitoring data, and the acquired or processed extension device data, and transmit the target visual information to the target device through the communication assembly 150.

Exemplarily, when the second processor 130 is in the working state, the second processor 130 is further configured to obtain feedback information transmitted by the target device in response to the extension device data.

In some embodiments, when the second processor 130 is in the working state, the first processor 120 is also in the working state, so as to achieve coordination between the defibrillation function, the monitoring function and the network function based on the first processor 120, and the extension function based on the second processor 130.

Exemplarily, the second processor 130 is further configured to transmit the acquired extension device data and/or extension monitoring data to the first processor 120; or the second processor 130 is further configured to process the acquired extension device data and/or extension monitoring data and then transmit the processed extension device data and/or extension monitoring data to the first processor 120. For example, the second processor 130 transmits acquired ultrasound image data and/or laryngoscope image data to the first processor 120.

Exemplarily, the second processor 130 is further configured to process ultrasound data and/or laryngoscope data to generate ultrasound image data and/or laryngoscope image data. For example, when the extension device 10 mainly includes a separate acquisition device, processing is required to obtain image data. For example, the second processor 130 processes an ultrasonic echo signal acquired from the ultrasonic device 11 to generate ultrasonic image data based on the ultrasonic echo signal, and transmits the ultrasonic image data to the first processor 120.

In some embodiments, the first processor 120 obtains defibrillation data through the defibrillation assembly 110, and/or obtains monitoring data through the monitoring assembly 140, and/or performs a defibrillation task through the defibrillation assembly 110, according to the extension device data and/or the extension monitoring data.

For example, the extension device data and/or the extension monitoring data may be used to indicate a patient state. For example, when the extension device data and/or the extension monitoring data indicate that the patient state is suitable for defibrillation, the defibrillation assembly 110 performs a defibrillation task.

For example, the extension device data can be configured to indicate a patient state to medical staff. For example, when the extension device data indicates that the medical staff is performing an ultrasound diagnosis on the patient, the first processor 120 automatically starts to acquire monitoring data through the monitoring assembly 140 according to the extension device data, and can also perform a defibrillation task through the defibrillation assembly 110 after the ultrasound diagnosis is completed.

In some implementations, the first processor 120 is further configured to transmit to the target device through the communication assembly 150, the data transmitted by the second processor 130. For example, the extension device data and/or the extension monitoring data transmitted by the second processor 130 is transmitted to the target device, and the target device feeds back a corresponding control instruction; the first processor 120 can control the defibrillation assembly to perform the defibrillation task according to the control instruction.

In some implementations, the first processor 120 may also output data transmitted by the second processor 130 through the human-machine interaction assembly 160. For example, when the second processor 130 is in the working state, the second processor 130 is also configured to transmit ultrasound image data and/or laryngoscope image data to the first processor 120, so as to display an ultrasound image and/or a laryngoscope image through the human-machine interaction assembly 160.

Exemplarily, the second processor 130 transmits the extension device data and/or the extension monitoring data to the first processor 120, and the first processor 120 displays the extension device data and/or the extension monitoring data together with the monitoring data and/or the defibrillation data through the human-machine interaction assembly 160. When the second processor 130 malfunctions or the extension device 10 malfunctions, an ability of the first processor 120 to display information (such as defibrillation data and monitoring data) is not affected, thereby having a high security.

In some embodiments, when the first processor 120 outputs the data transmitted by the second processor 130 through the human-machine interaction assembly 160, the data transmitted by the second processor 130, such as an ultrasound image and/or a laryngoscope image, can be superimposed and displayed on a partial display area of a display interface for the defibrillation data and/or the monitoring data. There is no need to perform data fusion processing on the ultrasound image and/or the laryngoscope image with the defibrillation data and/or the monitoring data, and the ultrasound image and/or the laryngoscope image can be displayed directly with a high real-time performance and a good security, thus preventing delays and freezes in the displayed image. The data transmitted by the second processor 130 is displayed in a window or picture-in-picture manner, so that the user can further analyze a patient state based on the data transmitted by the second processor 130 when viewing the defibrillation data and/or the monitoring data.

In other embodiments, the first processor 120 generates target visual information according to the defibrillation data and/or the monitoring data and/or data transmitted by the second processor 130; and the human-machine interaction assembly 160 is configured to output the target visual information. It should be noted that the target visual information is used for display on a same display interface. For example, the first processor 120 fuses the defibrillation data and/or the monitoring data with the data transmitted by the second processor 130 to obtain image data to be displayed. The fusion can be hardware fusion or software fusion. For example, the first processor 120 is configured to generate a target image, according to the monitoring data and the extension device data and/or the extension monitoring data transmitted by the second processor 130, and output the target image through the human-machine interaction assembly 160. Optionally, different areas of the target image are a respective area of the defibrillation data and/or the monitoring data, a respective area of the extension device data and/or the extension monitoring data; or the extension monitoring data and/or the extension device data are superimposed on an image of the defibrillation data or the monitoring data, so as to obtain a target image. Of course, it is not limited thereto. For example, the first processor 120 may display the defibrillation data, the monitoring data, the extension device data, and the extension monitoring data, in a time-sharing manner through a display assembly.

In some embodiments, when the human-machine interaction assembly 160 is connected with the first processor 120, the human-machine interaction assembly 160 can implement at least part of human-machine interaction function corresponding to the defibrillation function and the monitoring function, and can also implement at least part of human-machine interaction function of the extension device 10, which is convenient for user operation. Of course, it is not limited to this. For example, the human-machine interaction function of the extension device 10 and the human-machine interaction function corresponding to the defibrillation function and the monitoring function can also be implemented based on different human-machine interaction assemblies 160.

Exemplarily, when the second processor 130 is in the working state, the first processor 120 is further configured to acquire an operation instruction for the second processor 130 through the human-machine interaction assembly 160, and transmit the operation instruction to the second processor 130; the second processor 130 is configured to process the extension device data acquired from the extension device 10 according to the operation instruction.

Exemplarily, when the second processor 130 is in the working state, the first processor 120 is further configured to acquire an operation instruction for the extension device 10 through the human-machine interaction assembly 160, and transmit the operation instruction for the extension device 10 to the second processor 130; the second processor 130 is configured to control the extension device 10 to perform a corresponding extension task according to the operation instruction. The extension device 10 in this example may be a complete device, for example, a complete ultrasound device capable of generating an ultrasound image based on an ultrasound echo signal acquired by an acquisition device.

For example, the first processor 120 is configured to determine whether the operation instruction acquired through the human-machine interaction assembly 160 is an operation instruction for the extension device 10, and transmit the operation instruction for the extension device 10 to the second processor 130. Of course, this disclosure is not limited thereto. For example, the first processor 120 may transmit all operation instructions acquired through the human-machine interaction assembly 160 to the second processor 130, and the second processor 130 determines whether any operation instruction is an operation instruction for the extension device 10.

Exemplarily, a display assembly of the human-machine interaction assembly 160 provides a first operation interface/area and a second operation interface/area, and the first processor 120 can control the display assembly to display the first operation interface/area or the second operation interface/area according to a user operation on the human-machine interaction assembly 160. When the user enters an operation instruction on/in the second operation interface/area, the acquired operation instruction is transmitted to the second processor 130, and when the user enters an operation instruction in/on the first operation interface/area, the operation instruction is determined as not an operation instruction for the extension device 10.

Exemplarily, the display assembly can be configured to display a first display interface/area and a second display interface/area. Optionally, the first display interface/area can be configured to display monitoring data and/or defibrillation data, and the second display interface/area can be configured to display extension device data, such as ultrasound image data, etc. When the first processor 120 detects an operation in the second area, an acquired operation instruction may be transmitted to the second processor 130.

Exemplarily, the operation instruction for the extension device 10 is used to indicate the extension device 10 to be in a turn-on state or a turn-off state, such as to indicate the second processor 130 to start acquiring an ultrasound image through the ultrasound device 11, and indicate the second processor 130 to adjust an imaging mode (such as continuous Doppler mode, pulsed Doppler mode) and an imaging parameter (such as Doppler frequency) of the ultrasound device 11. Of course, it is not limited to this, for example, the operation instruction is used to indicate to adjust a brightness of a light source at the laryngoscope device 12. When the second processor 130 malfunctions or the extension device 10 malfunctions, abilities of the first processor 120 are not affected, which abilities includes acquiring respective operation instruction of the defibrillation function and the monitoring function through the human-machine interaction assembly 160, and according to the operation instruction, obtaining the defibrillation data through the defibrillation assembly 110 and/or obtaining the monitoring data through the monitoring assembly 140 and/or performing a defibrillation task through the defibrillation assembly 110.

Exemplarily, the first processor 120 is configured to acquire an operation instruction for the second processor 130 through the human-machine interaction assembly 160, and transmit the operation instruction to the second processor 130; the second processor 130 processes the obtained extension device data according to the operation instruction. Optionally, the extension device 10 in this example only includes an acquisition device, such as an ultrasound probe. The second processor 130 generates an ultrasonic image based on the ultrasonic echo signal acquired by the acquisition device according to the operation instruction.

Exemplarily, the first processor 120 generates target visual information based on the defibrillation data and/or the monitoring data and/or data transmitted by the second processor 130, and transmits the generated target visual information to the second processor 130; the second processor 130 transmits the target visual information to a target device through the extension communication assembly. For example, a target image displayed by the first processor 120 through the human-machine interaction assembly 160 is consistent with a target image displayed by the target device, so as to better adapt to a user habit.

Exemplarily, the second processor 130 is further configured to transmit the acquired or processed extension device data to the target device via an extension communication assembly or a communication assembly 150. For example, the second processor 130 is further configured to transmit ultrasound image data and/or laryngoscope image data to the target device through the communication assembly 150. The target device and/or a user of the target device may give a remote guidance based on the extension device data, such as ultrasound image data.

The second processor 130 is further configured to transmit the acquired or processed extension device data to the target device via the extension communication assembly or the communication assembly 150. For example, the second processor 130 is configured to generate target visual information according to the defibrillation data and/or the monitoring data and the acquired or processed extension device data, and transmit the target visual information to the target device through the extension communication assembly or the communication assembly 150.

In some embodiments, a processor state of the second processor 130 can be switched to the working state, and a starting time required for the first processor 120 to switch from a turn-off state to a working state is less than a starting time required for the second processor 130 to switch from a turn-off state to a working state. The second processor 130 uses a processor with stronger performance, and the starting time required to switch to the working state is longer. In the embodiment of this disclosure, the first processor 120 is switched from the turn-off state to the working state when the defibrillation device 100 is started, and the second processor 130 is maintained in the turn-off state when the defibrillation device 100 is started, so as to prevent the time for the second processor 130 to be switched to the working state from affecting a rapid starting of the defibrillation device 100 and an execution of basic defibrillation task.

In some embodiments, the second processor 130 is switched to the working state according to at least one of: at least one of the extension device 10, the extension function assembly 20 and the extension monitoring assembly 30 satisfies a first preset extension start condition; the defibrillation data and/or the monitoring data satisfy/satisfies a second preset extension start condition, and the monitoring data is obtained by the first processor 120 through processing data acquired from the monitoring assembly 140; a processor start instruction and/or an extension start instruction are/is acquired through the human-machine interaction assembly 160; wherein the processor start instruction is configured to control the second processor 130 to be switched to the working state, and the extension start instruction is configured to control the second processor 130 to be switched to the working state and to acquire the extension device data from the extension device 10 which device corresponds to the extension start instruction. In some embodiments, the second processor 130 may also be switched to the working state at a fixed time. If the above conditions are not satisfied within a certain time period, the second processor 130 may be switched to the turn-off state or the low power consumption state.

Exemplarily, the first processor 120 is capable of switching a processor state of the second processor 130. For example, the first processor 120 can switch the second processor 130 from a turn-off state or a low power consumption state to a working state, or can switch the second processor 130 from a working state to the turn-off state or the low power consumption state. Of course, it is not limited to this. For example, a processor state of the second processor 130 can be controlled to be switched between a turn-off state and a low power consumption state. Exemplarily, the first processor 120 may transmit a control information to the second processor 130, so as to control the processor state of the second processor 130 to be switched.

Optionally, the defibrillation device 100 further includes a third processor, which is a processor other than the first processor 120 and the second processor 130; the third processor is capable of switching a processor state of the second processor 130. For example, the third processor of the defibrillation device 100 is connected with the second processor 130, and may also be connected with the first processor 120, or may also be connected with the human-machine interaction assembly 160. For example, the third processor can switch the second processor 130 from a turn-off state or a low power consumption state to a working state, or can switch the second processor 130 from a working state to a turn-off state or a low power consumption state. Of course, it is not limited to this. For example, a processor state of the second processor 130 can be controlled to be switched between a turn-off state and a low power consumption state. Exemplarily, the third processor may transmit a control information to the second processor 130, so as to control the processor state of the second processor 130 to be switched.

Optionally, the second processor 130 can switch a processor state of the second processor 130. Exemplarily, when the processor state of the second processor 130 is in a low power consumption state or a working state, the processor state of the second processor 130 can be switched.

For example, when the second processor 130 is in the low power consumption state, the second processor 130 can be switched to a turn-off state or a working state; or when the second processor 130 is in a working state, the second processor 130 can be switched to a turn-off state or a low power consumption state.

For example, when the second processor 130 is in a low power consumption state, the second processor 130 can also independently decide whether to switch to a turn-off state or a working state. For example, the second processor 130 has some data transmission capabilities and information processing capabilities when in a low power consumption state. For example, preset information, such as defibrillation data and/or monitoring data, can be transmitted to the first processor 120, wherein the monitoring data is obtained by the first processor 120 through processing the data acquired from the monitoring assembly 140. Of course, it is not limited to this. For example, the second processor 130 can also determine the extension device 10 which is connected with the defibrillation device 100 when in a low power consumption state, and/or can acquire a human-machine interaction instruction through the human-machine interaction assembly 160. When the second processor 130 is in a low power consumption state, the second processor 130 can independently decide whether to switch to a working state or a turn-off state according to at least one of: preset information acquired from the first processor 120, an extension device 10 connected with the defibrillation device 100, and a human-machine interaction instruction.

In some implementations, the processor state of the second processor 130 can be switched from a turn-off state or a low power consumption state to a working state. For example, the first processor 120 or the third processor can switch the processor state of the second processor 130 from the turn-off state or the low power consumption state to the working state; or the second processor 130 in the low power consumption state can switch the processor state of the second processor 130 to the working state.

Exemplarily, a first extension start condition is configured to indicate device(s) and/or component(s) which is/are required for the second processor 130 to switch to the working state. For example, at least one of the extension device 10, the extension function assembly 20 and the extension monitoring assembly 30 satisfying a first preset extension start condition, includes: the extension device 10 and/or the extension monitoring assembly 30 are/is connected with the defibrillation device; for example, at least one of the extension function assembly 20 or the extension monitoring assembly 30 is required to be connected with the defibrillation device, or a preset number and type of extension device 10 or extension monitoring assembly 30 is required to be connected with the defibrillation device. As another example, at least one of the extension device 10, the extension function assembly 20 and the extension monitoring assembly 30 satisfying a first preset extension start condition, includes: the extension device 10, the extension function assembly 20, and the extension monitoring assembly 30 are connected with the defibrillation device 100 and are in a working state. For example, when the defibrillation device 100 is connected with the ultrasound device 11 and/or the laryngoscope device 12 and the ultrasound device 11 and/or the laryngoscope device 12 are in the working state, the processor state of the second processor 130 is switched to the working state, so as to enable the second processor 130 to obtain ultrasound image data and/or laryngoscope image data, and further to transmit the obtained ultrasound image data and/or laryngoscope image data to the first processor 120. When the defibrillation device 100 is not connected with some or all extension devices 10 which correspond to the first extension start condition, the processor state of the second processor 130 may not be switched to the working state, for example, the processor state of the second processor 130 may be switched from the low power consumption state to the turn-off state. By controlling the second processor 130 to be turned on or turned off, or to sleep as needed, the power consumption of the defibrillation device 100 may be effectively reduced.

For example, the first processor 120 or the third processor is configured to switch the processor state of the second processor 130 to the working state, when the extension device 10 satisfies the first preset extension start condition. For example, the second processor 130 in the low power consumption state is configured to switch the processor state of the second processor 130 to the working state, when the extension device 10, which is connected with the defibrillation device 100, satisfies the first preset extension start condition.

Exemplarily, the processor state of the second processor 130 is switched according to the defibrillation data and/or the monitoring data; wherein the monitoring data is obtained by the first processor 120 through processing the data acquired from the monitoring assembly 140.

Optionally, the processor state of the second processor 130 is switched to the working state, when the defibrillation data and/or the monitoring data satisfy/satisfies a second preset extension start condition. For example, the first processor 120 or the third processor is configured to switch the processor state of the second processor 130 to the working state, when the defibrillation data and/or the monitoring data satisfy/satisfies the second preset extension start condition. For example, the second processor 130 in the low power consumption state is configured to switch the processor state of the second processor 130 to the working state, when the defibrillation data and/or the monitoring data satisfy/satisfies the second preset extension start condition.

Exemplarily, the defibrillation data and/or the monitoring data satisfying the second preset extension start condition, includes: a patient is determined to need a diagnosis by the extension device 10 or the extension monitoring assembly 30, according to the defibrillation data and/or the monitoring data. Optionally, when a patient is determined to need a diagnosis by the extension device 10, such as an ultrasound device and a laryngoscope, according to electrocardiogram, blood oxygen, and blood pressure of the patient, the processor state of the second processor 130 can be switched to the working state, so as to enable the second processor 130 to acquire ultrasound image data and/or laryngoscope image data. When the patient is determined not to need a diagnosis by the extension device 10, it is not necessary to switch the processor state of the second processor 130 to the working state. For example, the processor state of the second processor 130 can be switched from a low power consumption state to a turn-off state. By controlling the second processor 130 to be turned on or turned off, or to sleep as needed, the power consumption of the defibrillation device 100 may be effectively reduced.

Exemplarily, the processor state of the second processor 130 is switched to the working state, when a processor start instruction and/or an extension start instruction is acquired through the human-machine interaction assembly 160. That is, the second processor 130 can be switched to the working state according to the processor start instruction and/or the extension start instruction, wherein the processor start instruction and/or the extension start instruction is acquired through the human-machine interaction assembly 160. Wherein the processor start instruction is configured to control the second processor 130 to be switched to the working state, and the extension start instruction is configured to control the second processor 130 to be switched to the working state and to acquire extension device data from the extension device 10 which extension device corresponds to the extension start instruction.

For example, the first processor 120 or the third processor is configured to acquire a processor start instruction and/or an extension start instruction through the human-machine interaction assembly 160, and switch the processor state of the second processor 130 to the working state according to the processor start instruction and/or the extension start instruction. For example, the second processor 130 in the low power consumption state can obtain a processor start instruction and/or an extension start instruction through the human-machine interaction assembly 160, and switch the processor state of the second processor 130 to the working state according to the processor start instruction and/or the extension start instruction.

The user can determine whether to start the extension device 10 according to a patient state, and trigger the processor start instruction and/or the extension start instruction through the human-machine interaction assembly 160 when needed, so as to switch the second processor 130 to the working state.

For example, after the second processor 130 is switched to the working state according to the processor start instruction, the second processor 130 can determine the extension device 10 which is connected with the defibrillation device 100, and the human-machine interaction assembly 160 outputs device information of the extension device 10 which is connected with the defibrillation device 100, then the corresponding extension device 10 can be further controlled to start according to an extension start instruction triggered by a user, so as to enable the second processor 130 to acquire the extension device data of the extension device 10 which extension device corresponds to the extension start instruction. Of course, it is not limited to this. For example, after the second processor 130 is switched to the working state, the extension device data can be obtained from all the extension devices 10. Alternatively, the first processor 120, or the second processor 130 in a low power consumption state may control the human-machine interaction assembly 160 to output device information of the extension device 10 connected with the defibrillation device 100, and may switch the processor state of the second processor 130 to the working state and control the corresponding extension device 10 to start, according to an extension start instruction triggered by a user.

Optionally, the human-machine interaction assembly 160 is also configured to output indication information when the defibrillation data and/or the monitoring data satisfy/satisfies a preset indication condition, and the indication information is used to indicate a user to operate to trigger the processor start instruction and/or the extension start instruction; wherein the monitoring data is obtained by the first processor 120 through processing the data acquired from the monitoring assembly 140. For example, the first processor 120, or the third processor, or the second processor 130 in the low power consumption state is also configured to output indication information through the human-machine interaction assembly 160, when the defibrillation data and/or the monitoring data satisfy/satisfies the preset indication condition, and the indication information is used to indicate a user to operate to trigger the processor start instruction and/or the extension start instruction.

For example, when a state of a patient is determined to need a further diagnosis by the extension device 10, such as an ultrasound device and a laryngoscope, according to electrocardiogram, blood oxygen, and blood pressure of the patient, indication information is outputted, and the user determines whether to start the second processor 130 and the corresponding extension device 10.

In some implementations, the processor state of the second processor 130 can be switched from the working state to the turn-off state or the low power consumption state. Exemplarily, when the second processor 130 is in the turn-off state or the low power consumption state, the second processor 130 stops acquiring the extension device data from the extension device 10 or the extension function assembly 20, and/or stops acquiring the monitoring data from the extension monitoring assembly 30.

In some embodiments, when the second processor 130 malfunctions, the defibrillation device stops acquiring the extension device data from the extension device 10 or the extension functional assembly 20, and/or stops acquiring the monitoring data from the extension monitoring assembly 30. That is, the functions of the second processor 130 can only be executed by the second processor 130 itself. When the second processor 130 malfunctions, the first processor 120 cannot replace the second processor 130 to execute its corresponding functions. Although an unidirectional or a bidirectional functional backup between the first processor 120 and the second processor 130 seems to be able to improve the system stability, however this means that the complexity of the circuit design is greatly increased. For example, interfaces of various extension devices need to be electrically connected not only with the second processor 130, but also with the first processor 120. As a result, not only the cost is increased, but also the stability and reliability of the system are affected.

The second processor 130 being in the turn-off state or the low power consumption state can reduce the power consumption of the defibrillation device 100 and can also reduce the influence on the first processor 120, thereby improving the reliability of the first processor 120 when performing a defibrillation task.

Exemplarily, when the first processor 120 malfunctions, the defibrillation assembly 110 stops performing the defibrillator task. It is understandable that the task that the first processor 120 can perform in the working state, such as the defibrillator task, the monitoring task, and the communication task, cannot be performed, that is, the first processor 120 is determined to malfunction. Similarly, the functions of the first processor 120 can only be performed by the first processor 120 itself. When the first processor 120 malfunctions, the second processor 130 cannot replace the first processor 120 to perform its corresponding function. The benefits are as described above and will not be repeated here.

In some implementations, the processor state of the second processor 130 is switched to the turn-off state or the low power consumption state, when the first processor 120 malfunctions. When the first processor 120 malfunctions, it cannot perform the basic defibrillation task. The power consumption of the defibrillation device can be reduced by switching the second processor 130 to the turn-off state or the low power consumption state.

For example, the second processor 130 can also obtain the processor state of the first processor 120, and switch the processor state of the first processor 120 to the turn-off state or the low power consumption state when the first processor 120 malfunctions. Optionally, the second processor 130 in the working state can also obtain a malfunction cause of the first processor 120, when the first processor 120 malfunctions, and can be switched to the turn-off state or the low power consumption state, when the malfunction cause is caused by the second processor 130, the extension device 10, the extension function assembly 20 or the extension monitoring assembly 30, or the malfunction cause is unknown. On the one hand, the power consumption of the defibrillation device can be reduced, and when the first processor 120 malfunctions due to interference from the second processor 130, the extension device 10, the extension function assembly 20, or the extension monitoring assembly 30, the first processor 120 can resume normal operation, thereby restoring the basic defibrillation function.

Exemplarily, the first processor 120 or the third processor switches the processor state of the second processor 130 from the working state to the turn-off state or the low power consumption state; or the second processor 130 in a working state can switch the processor state of the second processor 130 to the turn-off state or the low power consumption state.

Exemplarily, the processor state of the second processor 130 is switched to the turn-off state or the low power consumption state, so as to reduce the power consumption of the defibrillation device 100, when at least one of the extension device 10, the extension function assembly 20 and the extension monitoring assembly 30 satisfies a first preset extension turn-off condition, and/or the defibrillation data and/or the monitoring data satisfy a second preset extension turn-off condition.

Optionally, the processor state of the second processor 130 is switched to the turn-off state or the low power consumption state, when at least one of the extension device 10, the extension function assembly 20 and the extension monitoring assembly 30 satisfies a first preset extension turn-off condition. For example, when the defibrillation device 100 is not connected with the ultrasound device 11 and/or the laryngoscope device 12, the processor state of the second processor 130 is switched to the turn-off state or the low power consumption state; or when the defibrillation device 100 is not connected with some or all of the extension device(s) 10 which device(s) correspond(s) to the first extension start condition, the processor state of the second processor 130 is switched to the turn-off state or the low power consumption state.

Optionally, the processor state of the second processor 130 is switched to the turn-off state or the low power consumption state, when the defibrillation data and/or the monitoring data satisfy/satisfies a second preset extension turn-off condition. For example, the first processor 120 or the third processor is further configured to switch the processor state of the second processor 130 to the turn-off state or the low power consumption state when the defibrillation data and/or the monitoring data satisfy/satisfies a second preset extension turn-off condition. For example, the second processor 130 in a working state is configured to switch the processor state of the second processor 130 to the turn-off state or the low power consumption state, when the defibrillation data and/or the monitoring data satisfy/satisfies the second preset extension turn-off condition.

Optionally, when a patient is determined not to need a diagnosis by the extension device 10, such as an ultrasound device and a laryngoscope, according to electrocardiogram, blood oxygen, and blood pressure of the patient, the second processor 130 is switched to the turn-off state or the low power consumption state, so as to reduce the power consumption of the defibrillation device 100.

Exemplarily, the processor state of the second processor 130 is switched to the turn-off state or the low power consumption state according to a turn-off instruction, wherein the turn-off instruction is acquired through the human-machine interaction assembly 160. For example, when a turn-off instruction is acquired through the human-machine interaction assembly 160, the first processor 120 or the third processor is further configured to switch the processor state of the second processor 130 to the turn-off state or the low power consumption state according to the turn-off instruction. For example, when a turn-off instruction is acquired through the human-machine interaction assembly 160, the second processor 130 in the working state is further configured to switch the processor state of the second processor 130 to the turn-off state or the low power consumption state according to the turn-off instruction.

The user can determine whether to start the extension device 10 according to a patient state, and trigger a turn-off instruction through the human-machine interaction assembly 160 when not needed to switch the second processor 130 to the turn-off state or the low power consumption state.

Optionally, when a state of a patient is determined to need a diagnosis by the extension device 10, such as an ultrasound device and a laryngoscope, according to electrocardiogram, blood oxygen, and blood pressure of the patient, the human-machine interaction assembly 160 can also output a corresponding suggestion for indication, and the user can determine whether to start the extension device 10 based on the indication and the state of the patient.

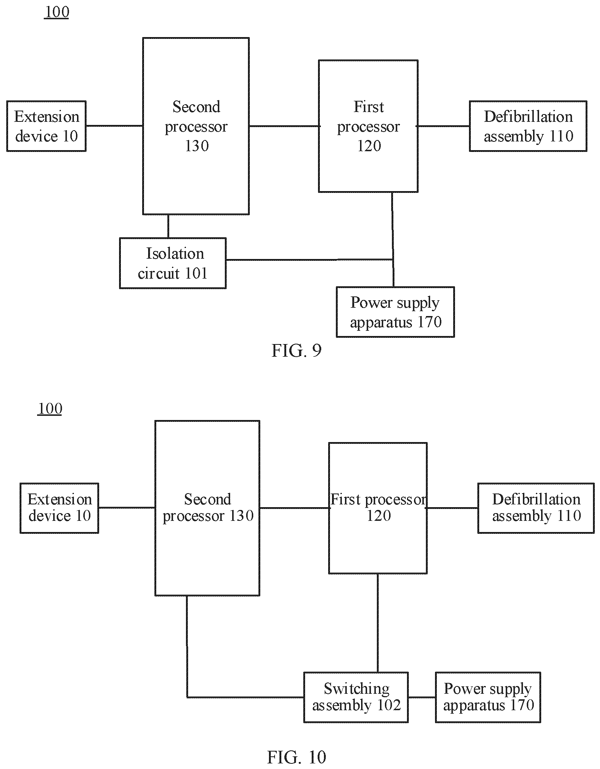

In some embodiments, referring to FIGS. 9 and 10, the defibrillation device 100 further includes a power supply apparatus 170.

Exemplarily, as shown in FIG. 9, a power supply terminal of the power supply apparatus 170 is connected with a power receiving terminal of the first processor 120 and a power receiving terminal of the second processor 130, and an isolation circuit 101 is provided between the power receiving terminal of the first processor 120 and the power receiving terminal of the second processor 130, which can prevent a malfunction of the second processor 130, such as a short circuit, from having a negative influence on the function of the first processor 120.

Exemplarily, the isolation circuit 101 is configured to disconnect the power supply for the second processor 130 while maintaining the power supply to the first processor 120, when a first preset condition occurs.

For example, the isolation circuit 101 is configured to disconnect the power supply to the second processor 130 when at least one of the second processor 130, the extension device 10, the extension functional assembly 20, and the extension monitoring assembly 30 malfunctions; and/or, the isolation circuit 101 is configured to disconnect the power supply to the second processor 130, when a supplied power of the power supply apparatus 170 is lower than a set value. In this way, the negative influence on the function of the first processor 120 caused by malfunctions of the second processor 130, the extension device 10, the extension function assembly 20, and the extension monitoring assembly 30, such as short circuit, or insufficient power, can be prevented, and power requirements of the defibrillation function, the monitoring function, and the human-machine interaction function can be prioritized.

Optionally, the isolation circuit 101 may also be configured to disconnect the power supply to the second processor 130, when the processor state of the second processor 130 needs to be switched to the turn-off state. The power consumption of the defibrillation device can be reduced, and the influence of the second processor 130, the extension device 10, the extension function assembly 20, and the extension monitoring assembly 30 on the function of the first processor 120 can be reduced.

Exemplarily, as shown in FIG. 10, the power supply apparatus 170 includes a first power supply terminal and a second power supply terminal, wherein the first power supply terminal is connected with a power receiving terminal of the first processor 120, and the second power supply terminal is connected with a power receiving terminal of the second processor 130. By supplying power to the first processor 120 and the second processor 130 respectively through different power supply terminals, it is possible to prevent a malfunction of the second processor 130, such as a short circuit, from having a negative influence on the function of the first processor 120.

Exemplarily, as shown in FIG. 10, the power supply apparatus 170 further includes a switching assembly 102, which is connected with the first power supply terminal and the second power supply terminal; the switching assembly 102 is configured to disconnect a connection with the second power supply terminal while maintaining a connection with the first power supply terminal, when a first preset condition occurs.

For example, the switching assembly 102 is configured to disconnect the power supply to the second processor 130, when at least one of the second processor 130, the extension device 10, the extension function assembly 20, and the extension monitoring assembly 30 malfunctions; and/or the switching assembly 102 is configured to disconnect the power supply to the second processor 130, when a supplied power of the power supply apparatus 170 is lower than a set value. In this way, the negative influence on the function of the first processor 120 caused by malfunctions of the second processor 130, the extension device 10, the extension function assembly 20, and the extension monitoring assembly 30, such as short circuit, or insufficient power, can be prevented, and power requirements of the defibrillation function, the monitoring function, and the human-machine interaction function can be prioritized.

The defibrillation device provided in an embodiment of this disclosure includes a defibrillation assembly, a first processor and a second processor; wherein the defibrillation assembly is configured to perform a defibrillation task; the first processor is configured to acquire data from the defibrillation assembly and processes said data to obtain defibrillation data; the second processor is configured to acquire extension device data from an extension device; the second processor is connected with the first processor, the second processor and the first processor are capable of transmitting preset information; after the defibrillation device is started, the first processor is in a working state by default, the second processor is in a turn-off state or a low power consumption state by default, wherein the second processor is capable of being switched to a working state. In this way, the defibrillation device is capable of quickly and completely starting to perform a basic defibrillation task and satisfying an emergency requirement of some scenarios. Furthermore, interference on the first processor and the defibrillation assembly from the second processor and the extension device can be prevented, which ensures a reliability of the defibrillation device in performing a defibrillation task.

It should be understood that the terminology used in the disclosure is for the purpose of describing particular embodiments only and is not intended to be limiting of the disclosure.

It will also be understood that the term "and/or" as used in the disclosure and the appended claims refers to and includes any and all possible combinations of one or more of the associated listed items.

The above are only specific implementation methods of this disclosure, but the protection scope of this disclosure is not limited thereto. Any technician familiar with the technical field can easily think of various equivalent modifications or substitutions within the technical scope disclosed in this disclosure, and these modifications or substitutions should be included in the protection scope of this disclosure. Therefore, the protection scope of this disclosure shall be based on the protection scope of the claims.

## Claims

1. A defibrillation device, **characterized in that**, comprising:
a defibrillation assembly, which is configured to perform a defibrillation task;
a first processor, which is configured to acquire data from the defibrillation assembly and process said data to obtain defibrillation data; and
a second processor, which is configured to acquire extension device data from an extension device;
wherein the second processor is connected with the first processor, the second processor and the first processor are capable of transmitting preset information;
after the defibrillation device is started, the first processor is in a working state by default, the second processor is in a turn-off state or a low power consumption state by default, wherein the second processor is capable of being switched to a working state.

2. The defibrillation device according to claim 1, **characterized in that**, further comprising a monitoring assembly; wherein the first processor is further configured to obtain data from the monitoring assembly and processes said data to obtain monitoring data;
wherein the monitoring assembly comprises at least one of: an electrocardiogram detection assembly, a blood oxygen detection assembly, and an NIBP detection assembly;
the defibrillation assembly comprises a defibrillation function assembly and/or a pacing function assembly.

3. The defibrillation device according to claim 1, **characterized in that**, further comprising a communication assembly, which is connected with the first processor;
wherein the first processor is further configured to establish a communicative connection with a target device through the communication assembly.

4. The defibrillation device according to claim 3, **characterized in that**, the first processor is further configured to transmit the defibrillation data and/or monitoring data to the target device through the communication assembly; the monitoring data is obtained by the first processor through processing data obtained from a monitoring assembly; and/or
the first processor is further configured to obtain control data from the target device through the communication assembly, and control the defibrillation assembly according to the control data.

5. The defibrillation device according to claim 1, **characterized in that**, further comprising:
an extension function assembly which is connected with the second processor; wherein the extension function assembly comprising at least one of: a data reading assembly, an extension human-machine interaction assembly, and a photographing assembly; wherein when the second processor is in the working state, the second processor is further configured to acquire the extension device data through at least one of the data reading assembly, the extension human-machine interaction assembly and the photographing assembly; and/or
an extension monitoring assembly, wherein when the second processor is in the working state, the second processor is further configured to acquire extension monitoring data from the extension monitoring assembly.

6. The defibrillation device according to claim 5, **characterized in that**, the second processor is further configured to transmit the extension device data and/or the extension monitoring data to the first processor; or the second processor is further configured to process the extension device data and/or the extension monitoring data and transmit the processed data to the first processor;
the first processor is further configured to transmit the data transmitted by the second processor to a target device through a communication assembly.

7. The defibrillation device according to any one of claims 1~6, **characterized in that**, when the defibrillation device has been completely started, the first processor is switched from a turn-off state to the working state, and the second processor is maintained in the turn-off state or is switched to the low power consumption state.

8. The defibrillation device according to any one of claims 1~6, **characterized in that**, a starting time, which is required for the first processor to be switched from a turn-off state to the working state, is less than a starting time, which is required for the second processor to be switched from the turn-off state to the working state.

9. The defibrillation device according to any one of claims 1~6, **characterized in that**, power consumption of the first processor in the working state is less than power consumption of the second processor in the working state; and/or
a performance parameter of the first processor is less than a performance parameter of the second processor.

10. The defibrillation device according to any one of claims 1~6, **characterized in that**, the second processor is capable of being selectively disassembled from the defibrillation device;
the defibrillation assembly is still capable of performing the defibrillation task after the second processor has been disassembled.

11. The defibrillation device according to claim 10, **characterized in that**, the first processor is assembled on a first circuit board, the second processor is assembled on a second circuit board;
wherein the second circuit board is capable of being selectively disassembled from the defibrillation device, and the defibrillation assembly is still capable of performing the defibrillation task after the second circuit board has been disassembled.

12. The defibrillation device according to any one of claims 1~6, **characterized in that**, when the first processor is in the working state, the second processor is in any one of: the turn-off state, the low power consumption state, and the working state; and
when the second processor is in the working state, the first processor is also in the working state.

13. The defibrillation device according to any one of claims 1~6, **characterized in that**, the second processor is switched to the working state according to at least one of:
at least one of the extension device, an extension function assembly, and an extension monitoring assembly satisfies a first preset extension start condition;
the defibrillation data and/or monitoring data satisfy/satisfies a second preset extension start condition, wherein the monitoring data is obtained by the first processor through processing data acquired from a monitoring assembly;
a processor start instruction and/or an extension start instruction are/is acquired through a human-machine interaction assembly; wherein the processor start instruction is configured to control the second processor to be switched to the working state; and the extension start instruction is configured to control the second processor to be switched to the working state and to acquire the extension device data from the extension device which extension device corresponds to the extension start instruction.

14. The defibrillation device according to claim 13, **characterized in that**,
at least one of the extension device, the extension function assembly, and the extension monitoring assembly satisfying a first preset extension start condition, comprises:
the extension device, the extension function assembly or the extension monitoring assembly is connected with the defibrillation device; or the extension device, the extension function assembly or the extension monitoring assembly is connected with the defibrillation device and in a working state;
the defibrillation data and/or monitoring data satisfying a second preset extension start condition, comprises:
a patient is determined to need a diagnosis by the extension device, the extension function assembly or the extension monitoring assembly, according to the defibrillation data and/or the monitoring data.

15. The defibrillation device according to any one of claims 1~14, **characterized in that**, when the first processor malfunctions, the defibrillation assembly stops performing the defibrillation task.

16. The defibrillation device according to any one of claims 1~14, **characterized in that**, when the first processor malfunctions, the defibrillation assembly stops acquiring the extension device data from the extension device or an extension function assembly, and/or stops acquiring monitoring data from an extension monitoring assembly.

17. A defibrillation device, **characterized in that**, comprising:
a defibrillation assembly, which is configured to perform a defibrillation task;
a first processor, which is configured to acquire data from the defibrillation assembly and process the data to obtain defibrillation data; and
a second processor, which is configured to acquire extension device data from an extension device and/or extension monitoring data from an extension monitoring assembly;
wherein the second processor is connected with the first processor, the second processor and the first processor are capable of transmitting preset information;
after the defibrillation device is started, the first processor is in a working state by default, the second processor is capable of being in a turn-off state, a low power consumption state or a working state; wherein, when the second processor does not acquire the extension device data or the extension monitoring data, a duration when the second processor is in the working state is less than a duration when the second processor is in the turn-off state and/or the low power consumption state.

18. A defibrillation device, **characterized in that**, comprising:
a defibrillation assembly, which is configured to perform a defibrillation task;
a first processor, which is configured to acquire data from the defibrillation assembly and process the data to obtain defibrillation data; and
a second processor, which is configured to acquire extension device data from an extension device;
wherein the second processor is connected with the first processor, the second processor and the first processor are capable of transmitting preset information;
after the defibrillation device is started, the first processor is in a working state by default, the second processor is capable of being in a turn-off state, a low power consumption state or a working state; wherein a duration when the second processor is in the working state is less than a duration when the first processor is in the working state.

19. The defibrillation device according to claim 17 or claim 18, **characterized in that**, further comprising a monitoring assembly; wherein the first processor is further configured to obtain data from the monitoring assembly and processes said data to obtain monitoring data;
wherein the monitoring assembly comprises at least one of: an electrocardiogram detection assembly, a blood oxygen detection assembly, and an NIBP detection assembly;
the defibrillation assembly comprises a defibrillation function assembly and/or a pacing function assembly.

20. The defibrillation device according to claim 17 or claim 18, **characterized in that**, further comprising a communication assembly, which is connected with the first processor;
wherein the first processor is further configured to establish a communicative connection with a target device through the communication assembly.

21. The defibrillation device according to claim 20, **characterized in that**, the first processor is further configured to transmit the defibrillation data and/or monitoring data to the target device through the communication assembly; the monitoring data is obtained by the first processor through processing data obtained from a monitoring assembly; and/or
the first processor is further configured to obtain control data from the target device through the communication assembly, and control the defibrillation assembly according to the control data.

22. The defibrillation device according to claim 17 or claim 18, **characterized in that**, further comprising:
an extension function assembly which is connected with the second processor; wherein the extension function assembly comprising at least one of: a data reading assembly, an extension human-machine interaction assembly, and a photographing assembly; wherein when the second processor is in the working state, the second processor is further configured to acquire the extension device data through at least one of the data reading assembly, the extension human-machine interaction assembly and the photographing assembly; and/or
an extension monitoring assembly, wherein when the second processor is in the working state, the second processor is further configured to acquire extension monitoring data from the extension monitoring assembly.

23. The defibrillation device according to claim 22, **characterized in that**, the second processor is further configured to transmit the extension device data and/or the extension monitoring data to the first processor; or the second processor is further configured to process the extension device data and/or the extension monitoring data and transmit the processed data to the first processor;
the first processor is further configured to transmit the data transmitted by the second processor to a target device through a communication assembly.

24. The defibrillation device according to any one of claims 17~23, **characterized in that**, the second processor is capable of being switched to the working state;
wherein a starting time, which is required for the first processor to be switched from a turn-off state to the working state, is less than a starting time, which is required for the second processor to be switched from the turn-off state to the working state.

25. The defibrillation device according to any one of claims 17~23, **characterized in that**, power consumption of the first processor in the working state is less than power consumption of the second processor in the working state; and/or
a performance parameter of the first processor is less than a performance parameter of the second processor.

26. The defibrillation device according to any one of claims 17~23, **characterized in that**, the second processor is switched to the working state according to at least one of:
at least one of the extension device, an extension function assembly, and an extension monitoring assembly satisfies a first preset extension start condition;
the defibrillation data and/or monitoring data satisfy/satisfies a second preset extension start condition, wherein the monitoring data is obtained by the first processor through processing data acquired from a monitoring assembly;
a processor start instruction and/or an extension start instruction are/is acquired through a human-machine interaction assembly; wherein the processor start instruction is configured to control the second processor to be switched to the working state; and the extension start instruction is configured to control the second processor to be switched to the working state and to acquire the extension device data from the extension device which extension device corresponds to the extension start instruction.

27. The defibrillation device according to claim 26, **characterized in that**,
at least one of the extension device, the extension function assembly, and the extension monitoring assembly satisfying a first preset extension start condition, comprises:
the extension device, the extension function assembly or the extension monitoring assembly is connected with the defibrillation device; or the extension device, the extension function assembly or the extension monitoring assembly is connected with the defibrillation device and in a working state;
the defibrillation data and/or monitoring data satisfying a second preset extension start condition, comprises:
a patient is determined to need a diagnosis by the extension device, the extension function assembly or the extension monitoring assembly, according to the defibrillation data and/or the monitoring data.
